# EUROPÄISCHE PATENTSCHRIFT

(11) **EP 3 349 817 B1**
(45) Veröffentlichungstag und Bekanntmachung des Hinweises auf die Patenterteilung: **25.11.2020**
(21) Anmeldenummer: 16762972.4
(22) Anmeldetag: 06.09.2016
(51) Int. Cl.: A61M 1/00

(54) **WUNDVERSORGUNGSANORDNUNG**
WOUND DRESSING ASSEMBLY
SYSTÈME DE TRAITEMENT DE PLAIES

(30) Priorität: 17.09.2015 DE 202015006570 U
(43) Veröffentlichungstag der Anmeldung: 25.07.2018
(73) Patentinhaber: Lohmann & Rauscher GmbH, 2525 Schönau/Triesting (AT)
(72) Erfinder: LOSKE, Gunnar, 22926 Ahrensburg (DE)
(74) Vertreter: Seranski, Klaus
(86) Internationale Anmeldenummer: PCT/EP2016/001504
(87) Internationale Veröffentlichungsnummer: WO 2017/045749

(56) Entgegenhaltungen:
- WO-A1-2006/056294
- DE-A1-102011 121 072
- DE-U1-202013 002 253
- DE-U1-202013 008 544

## Beschreibung

Die Erfindung betrifft eine Wundversorgungsanordnung nach dem Oberbegriff des Patentanspruchs 1.

Entero-atmosphärische Fisteln bilden eine Verbindung zwischen einem Darmlumen und der Haut, über die sich der Darminhalt durch eine Hautöffnung entleeren kann. Die Fisteln entstehen als Komplikation im Verlauf von Abdominaloperationen. Im Verlauf der Behandlung kann der Darminhalt über die Fistel in einen Beutel abgeleitet werden. Gleichzeitig ergibt sich die Notwendigkeit, um die Fistelöffnung gebildete Wunden zu versorgen.

Wundversorgungsanordnungen nach dem Oberbegriff des Patentanspruchs 1 sind in der DE 20 2013 008 544 U1, der DE 20 2013 002 253 U1, der DE 10 2011 121 072 A1 und der WO 2006/056294 A1 angegeben.

Der Erfindung liegt die Aufgabe zugrunde, Wundversorgungsanordnungen bereitzustellen, mit denen einerseits eine wirkungsvolle Versorgung von im Bereich von Fistelöffnungen bestehenden Wunden und andererseits eine gewünschte Behandlung des Patienten unter Ausnutzung der Fistelöffnung gewährleistet werden kann.

Erfindungsgemäß wird diese Aufgabe durch die im kennzeichnenden Teil des Patentanspruchs 1 angegebene Weiterbildung von Wundversorgungsanordnungen der eingangs angegebenen Art gelöst.

Eine einseitig offenporige Folie bezeichnet dabei eine doppel- oder mehrlagige Folie, deren eine Begrenzungsfläche von einer durchgehenden, fluiddichten Folie gebildet wird und bei der ein zwischen einzelnen Folienlagen gebildeter Raum über eine in einer anderen Folienlage gebildeten Öffnung mit der Umgebung in Verbindung steht. Eine mit Poren ausgestattete Folienlage bildet dabei die offenporige Seite der Abdeckeinrichtung. Die Poren können als sogenannte dreidimensionale Poren ausgeführt sein, welche sich trichterförmig in einen zwischen den Folienlagen gebildeten Raum erstrecken. Wenn der zwischen den Folienlagen gebildete Raum über eine fluiddicht an die fluiddichte (äußere) Folienlage angeschlossene Leitung mit einer Unterdruckerzeugungseinrichtung in Verbindung steht, kann eine gleichmäßige Druckverteilung in dem zwischen der Abdeckeinrichtung und der Haut bzw. der Wunde gebildeten Raum über die einseitig offenporige Folie erfolgen. Diese gleichmäßige Druckverteilung fördert die Wundheilung in diesem Bereich.

Die Verstärkungseinrichtung einer erfindungsgemäßen Wundversorgungsanordnung kann einen Verstärkungsring, insbesondere Kunststoffring aufweisen, der im Behandlungszustand die Fistelöffnung vollständig umläuft und an dem die Abdeckeinrichtung fluiddicht angeschlossen ist. Dazu kann die Abdeckeinrichtung beispielsweise auf eine der Wunde abgewandten Begrenzungsfläche des Verstärkungsrings aufgeklebt sein. Der Verstärkungsring kann aus Polyvinyl, Polyurethan und/oder Silikon bestehen. Im Rahmen der Erfindung ist vorgesehen, den Verstärkungsring massiv vollvolumig aus Kunststoff zu fertigen. Dabei kann der Ring in einer axialen Schnittebene einen rechteckigen Querschnitt aufweisen. Bei einer anderen Ausführungsform der Erfindung ist vorgesehen, daß der Verstärkungsring einen auf die Haut auflegbaren Flanschbereich und einen sich senkrecht dazu, etwa parallel zur Ringachse erstreckenden Stutzenbereich aufweist, wobei die Abdeckeinrichtung vorzugsweise auf eine der Haut abgewandten Begrenzungsfläche des Flanschbereichs aufliegt und fluiddicht damit verbunden werden kann. Der Stutzenbereich erlaubt den Anschluss einer beispielsweise beutelförmigen Aufnahmeeinrichtung, in dem der Darminhalt und/oder Wundsekrete, Urin oder dergleichen, abgeleitet werden können.

Gemäß einer weiter bevorzugten Ausführungsform der Erfindung weist der Stutzenbereich an seinem dem Flanschbereich abgewandten axialen Ende einen sich radial nach außen erstreckenden Anschlussflansch auf. Der Verstärkungsring kann wundabgewandt (Außenfläche der Abdeckeinrichtung) mit der Abdeckeinrichtung auch als Konnektierungssystem für ein gebräuchliches Anus praeter Stomabeutelsystem mit einem entsprechenden Adapter gefertigt werden. Dadurch kann die Erfindung gleichzeitig wie eine Stomaandruckplatte und ein Vakuumsiegelverband benutzt werden.

Im Rahmen der Erfindung ist es weiter bevorzugt, wenn der Verstärkungsring zumindest abschnittweise verformbar ausgeführt ist. Dann kann sich der Verstärkungsring der Kontur der Wunde bzw. der Hautoberfläche im Bereich der Wunde anpassen. Dann wird durch Erzeugung eines Unterdrucks in dem von der Abdeckeinrichtung, der davon abgedeckten Haut und der Verstärkungseinrichtung begrenzten Raum die Haut- bzw. Wundoberfläche derart angelegt, daß der Raum auch im Bereich der Verstärkungseinrichtung in Form des verformbaren Verstärkungsrings durch Anlage des Verstärkungsrings an die Haut fluiddicht abgeschlossen ist.

Bei der zuletzt beschriebenen Ausführungsform der Erfindung kann der Verstärkungsring zumindest bereichsweise als von einer verformbaren Wand begrenzter Hohlkörper ausgeführt sein, der gegebenenfalls über eine Ventilanordnung mit einem Fluid befüllt werden kann. Bei dieser Ausführungsform kann der Verstärkungsring schlauchförmig mit einem mit Luft oder einem anderen Gas befüllbaren Innenlumen konstruiert werden. Dabei kann die Wand des schlauchförmigen Rings dünnwandig sein, um so eine gute Verformbarkeit sicherzustellen. Das Innenlumen des schlauchförmigen Rings kann fluidleitend mit einem Kanal verbunden sein, über den Luft, ein anderes Gas oder eine Flüssigkeit in das Innenlumen eingeführt und der Ring wie ein Schwimmring aufgepumpt werden kann. Der Kanal kann mit einem Ventil ausgerüstet sein, so daß das Gas bzw. die Flüssigkeit nicht oder nur kontrolliert entweichen kann. Durch die schwimmringartige Konstruktion kann zum einen der Ring der Wundauflagerung druckangepasst werden, zum anderen kann bei Entleerung ein kleineres Verpackungsmaß realisiert werden.

Das Ringprofil kann rundlich, kreisförmig, rechteckig oder dreieckig sein. Die wundseitige Kontaktfläche kann rundlich oder auch flächig sein. Wenn sie flächig ausgeformt ist, soll hiermit der Anpressdruck auf die Wundfläche gleichmäßiger verteilt werden, so daß keine Druckschäden entstehen. Vorzugsweise schließt das Ringprofil bündig mit der Abdeckeinrichtung ab. Dabei kann das Ringprofil die Abdeckeinrichtung wundnah und/oder wundfern überragen.

Die Behandlung einer Wunde im Bereich einer Fistelöffnung kann weiter verbessert werden, wenn eine zwischen der Abdeckeinrichtung und der Haut bzw. der Wunde anzuordnende, nachgiebige und fluidleitende Polsteranordnung vorgesehen ist, die die Öffnung zumindest abschnittweise umläuft und an die Verstärkungseinrichtung angeschlossen ist. Durch diese Weiterbildung der Erfindung kann bei Erzeugung eines Unterdrucks in dem zwischen der Verstärkungseinrichtung, der Abdeckeinrichtung und der Haut bzw. der Wunde gebildeten Raum ein besonders inniger Wundkontakt der Wundversorgungsanordnung über die Polsteranordnung im Bereich der Verstärkungseinrichtung bzw. der Öffnung erzielt werden. Dadurch wird die Abdichtung im Bereich des fistelnah auf der Wunde aufliegenden Verstärkungsrings und damit der Wundversorgungsanordnung insgesamt verbessert. Dann kann eine Vakuumversiegelung der Wundfläche vorgenommen werden, wobei andererseits die Entleerung von Fistelsekreten/Darminhalten in einen über der Öffnung angebrachten Beutel stattfinden kann.

Die Polsteranordnung kann zumindest abschnittweise als offenporiger Schaum, insbesondere Polyurethanschaum ausgeführt sein. Durch die dicht an dicht liegenden Poren des Polyurethanschaums kann eine festere Anhaftung auf der Wundfläche als mit einer offenporigen Folie erzeugt werden, so daß insbesondere in dem der Fistelöffnung angrenzenden Bereich eine innige Anbindung erzielt werden kann.

Die Polsteranordnung kann in nahezu beliebiger Form an der offenporigen Seite der einseitig offenporigen Folie anliegen. Dazu können Polsterstreifen, insbesondere Polyurethanschaumstreifen, einseitig mit einer Klebefläche ausgerüstet sein, so daß die Streifen mit dieser Klebefläche auf die offenporige Seite der einseitig offenporigen Folie aufgeklebt werden können. Dabei bleiben die Saugeigenschaften der offenporigen Einheit aus Folie und Polyurethanschaum unbeeinträchtigt.

Die Polsteranordnung kann einen die Verstärkungseinrichtung umlaufenden Polsterring und mindestens einen, vorzugsweise zwei, drei oder mehr sich ausgehend davon radial nach außen erstreckende Polsterarme aufweisen, wobei der Anschluss an die Unterdruckerzeugungseinrichtung im Bereich eines dem Polsterring abgewandten Endes eines der Polsterarme erfolgen kann.

Von der Öffnung der Abdeckeinrichtung, die in der Mitte der Abdeckfläche liegen soll, kann eine spinnennetzartige Struktur aus Polyurethanschaum ausgehen. Bei einer anderen Ausführungsform der Erfindung kann eine netzartig-strahlenförmige Anordnung mit fingerartigen Ausläufern vorgesehen sein. Durch eine solche Ausprägung der Polsteranordnung lässt sich eine gewisse Versteifung der Abdeckeinrichtung erreichen, die die flächige Ausbreitung und Platzierung der Abdeckeinrichtung auf der Wunde erleichtert.

Bei einer bevorzugten Ausführungsform der Erfindung ist die Abdeckeinrichtung zumindest abschnittsweise durchsichtig ausgeführt. Durch einen durchsichtigen Abschnitt der Abdeckeinrichtung lässt sich die Wundheilung beobachten. Ferner kann vorgesehen sein, daß die Polsteranordnung in einem durchsichtigen Bereich der Abdeckeinrichtung angeordnete buchstaben- und/oder ziffernförmige Markierungselemente aufweist. Die Markierungselemente scheinen lesbar durch die vorzugsweise folienartige Abdeckeinrichtung hindurch. Dadurch können beispielsweise Daten der Wundbehandlung in der Wundversorgungsanordnung selbst festgehalten werden oder das Wechseldatum für einen Verband der Wundversorgungsanordnung notiert werden. Die vorstehend erläuterte Art der Markierung und Beschriftung der Wundversorgungsanordnung kann auch mit nicht offenporigem Material, wie Stoffen oder Gasen vorgenommen werden. Schäume und Stoffe können eingefärbt sein.

Die im Rahmen der Erfindung einsetzbaren Streifen der Polsteranordnung, insbesondere Polyurethanstreifen, sollen bevorzugt eine Breite von 2 bis 10 mm haben und eine Höhe bis 10 mm aufweisen. Der direkt an der Verstärkungseinrichtung anliegende Polsterring bzw. diesen umlaufende bzw. davon abgehende Streifen sollen etwa so hoch wie das Ringprofil sein. Wenn dann Unterdruck an den offenporigen Polyurethanschaum angelegt wird, kollabiert dieser Schaum und führt so zu einem gewissen Ansaugen bzw. Andruck der Verstärkungseinrichtung an die fistelnahe Wundfläche.

Eine für die Versorgung von entero-atmosphärischen Fisteln ausgerüstete Wundversorgungsanordnung ist flächig wie ein Klebepflaster gestaltet. Sie weist vorzugsweise eine Fläche von 5 cm x 5 cm bis zu 50 cm x 50 cm auf. Die Öffnung der Abdeckeinrichtung ist vorzugsweise zentral platziert und ist besonders bevorzugt mit einem Verstärkungsring ausgestattet. Der Verstärkungsring kann einen Durchmesser von bis zu 15 cm aufweisen. An den Ring ist die vorzugsweise als einseitig offenporige Folie ausgeführte Abdeckeinrichtung fluiddicht angeschlossen. Dabei liegt die einseitig offenporige Folie an der Wunde an. Die entsprechende Fläche der einseitig offenporigen Folie kann mit zusätzlichem offenporigem Material, vorzugsweise offenporigem Polyurethanschaum flächig oder in Streifen oder in Schriftzügen ausgestattet sein. Diese Seite kann auch auf der intakten Haut direkt aufliegen. Im Randbereich kann dann die Abdeckeinrichtung mit einem Klebestreifen ausgestattet sein, der auf der gesunden Haut aufgeklebt ist. Diese Verklebung im Randbereich soll fluiddicht den Verband zum Rand hin abschließen.

Die erfindungsgemäße Wundversorgungsanordnung ist von besonderem Vorteil für eine Unterdruckbehandlung im Bereich entero-atmosphärischer Fisteln einsetzbar. Dabei können unterschiedliche offenporige Materialien (einseitige Folie/Schaum) in Kombination mit einer Verstärkungseinrichtung, insbesondere einem Verstärkungsring, der über eine Fistelöffnung gelegt werden soll, kombiniert werden. Es handelt sich um einen einseitig offenporigen Verband mit einer zentralen Öffnung, mit dem im Bereich einer Wunde mit einer Fistel mit einem vakuumerzeugenden System, vorzugsweise einer elektronischen Pumpe, ein Unterdruck erzeugt werden kann. Die Öffnung der Wundversorgungsanordnung kann je nach Bedarf für die Adaption für Anus praeter Stomabeutel konstruiert werden. Zweckmäßigerweise ist die Wundversorgungsanordnung überwiegend transparent, so daß vorzugsweise bei anliegender Wundversorgungsanordnung eine Wundbegutachtung erfolgen kann. Die Wundversorgungsanordnung kann direkt auf die Haut aufgeklebt werden. Ein besonderer Vorteil ist die Zeitersparnis und deutliche Vereinfachung der komplexen Wundversorgung derartiger Wunden.

Bei allen Ausführungsformen der Erfindung kann eine vorzugsweise beutelförmige Aufnahmeeinrichtung fest oder lösbar über der wundabgewandten Seite der Öffnung angebracht sein.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich Bezug genommen wird, erläutert. In der Zeichnung zeigt:
- Fig. 1: eine Wundversorgungsanordnung gemäß einer ersten Ausführungsform der Erfindung,
- Fig. 2: Schnittdarstellung der Wundversorgungsanordnung gemäß Fig. 1,
- Fig. 3: eine Wundversorgungsanordnung gemäß einer zweiten Ausführungsform der Erfindung, und
- Fig. 4: verschiedenartige Ausführungen einer Verstärkungseinrichtung erfindungsgemäßer Wundversorgungsanordnungen.

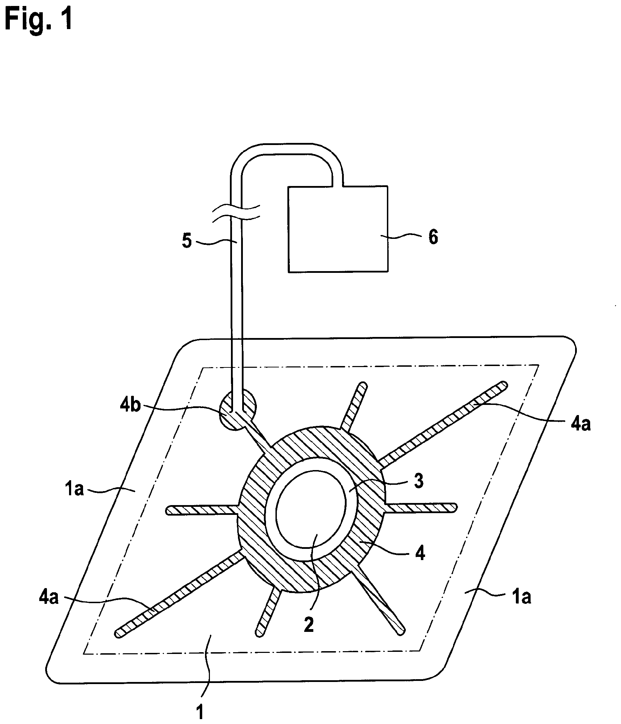

Figur 1 ist eine Aufsichtsdarstellung auf eine Abdeckeinrichtung in Form einer transparenten einseitig offenporigen Folie (1). Der umlaufende Rand (1a) ist mit einem Kleber ausgerüstet, mit der die Folie auf der Haut fixiert werden kann. In der Mitte der Folie befindet sich eine kreisförmige Öffnung (2). Die Öffnung wird von einem Kunststoffring (3) begrenzt, der mit der Folie nicht fluidleitend verschweißt ist. An den Ring angrenzend befindet sich auf der offenporigen Unterseite aufliegend ein offenporiger Polyurethanschaum (4), der sich sternenförmig mit fingerartigen Ausläufern (4a) über die Folie erstreckt. Der offenporige Polyurethanschaum ist fluidleitend mit der offenporigen Seite der Folie verbunden. Einer der fingerförmigen Ausläufer des Schaumes (4b) ist fluidleitend mit einem Schlauch (5) verbunden, dieser ist mit einem vakuumerzeugenden System (6) verbunden.

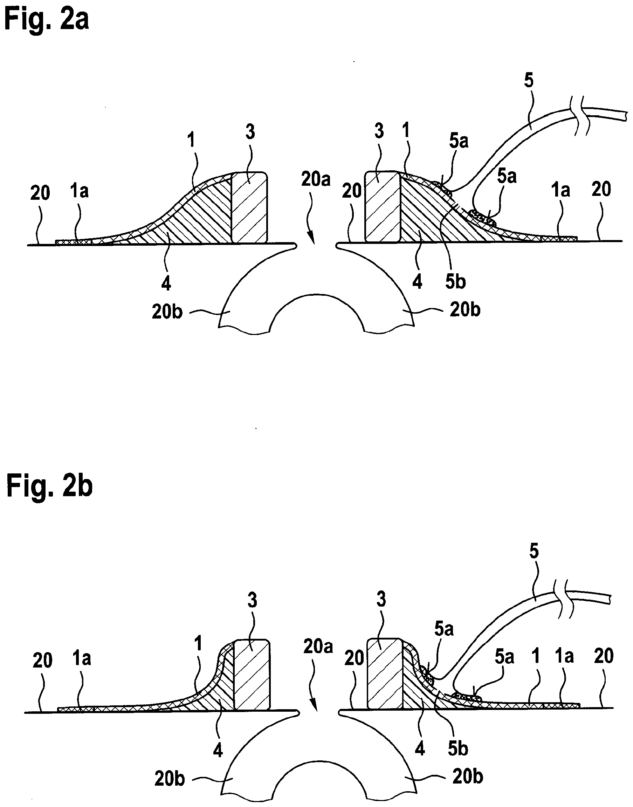

Figur 2a ist eine Querschnittsdarstellung von Figur 1 in Höhe der Ringöffnung. Die einseitig offenporige Folie (1) ist mit der offenporigen Unterseite auf eine Wunde (20), die eine Fistelöffnung (20a) zum Darm (20b) umschließt, aufgelegt. Im Randbereich der Folie (1a) ist die Folie aufgeklebt und schließt mit der Wunde oder Haut nicht fluidleitend ab. Über der Fistelöffnung (20a) ist der Ring (3) aufgesetzt. An dem Ring liegt eine fluidleitenden Polsteranordnung in Form eines offenporigen Polyurethanschaums an. Auf die Folie ist ein Schlauch (5) mit einer Klebefläche (5a) aufgeklebt. Über eine Öffnung (5b) ist der Schlauch fluidleitend mit der offenporigen Seite der Folie und mit dem offenporigen Schaum verbunden, so dass ein Vakuum angelegt werden kann.

Figur 2b ist die gleiche Querschnittdarstellung wie Figur 2a, mit dem Unterschied, dass über den Schlauch (5) ein Vakuum angelegt wurde und hierdurch der offenporige Polyurethanschaum (4) kollabiert.

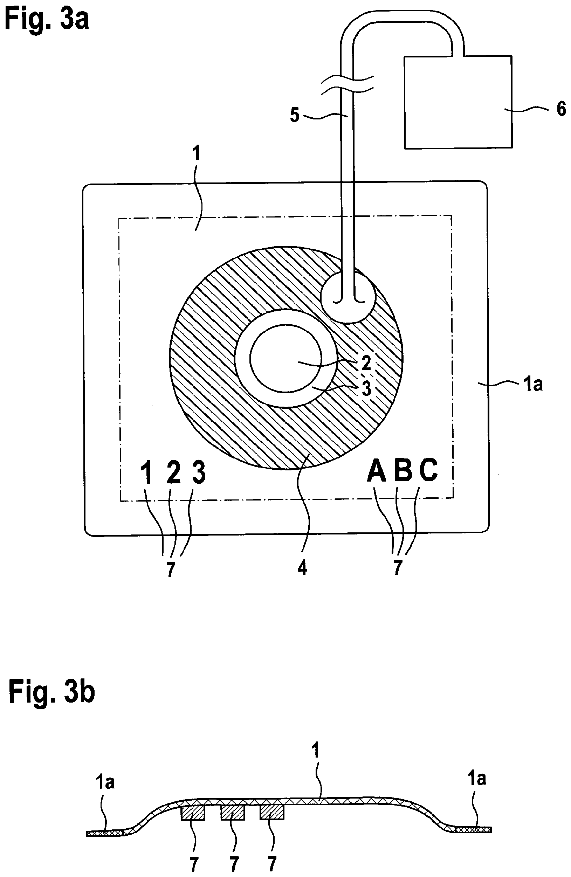

Figur 3a ist eine Aufsichtsdarstellung auf eine Abdeckeinrichtung in Form einer transparenten einseitig offenporigen Folie (1). Der umlaufende Rand (1a) ist mit einem Kleber ausgerüstet, mit der die Folie auf der Haut fixiert werden kann. In der Mitte der Folie befindet sich eine kreisförmige Öffnung (2). Die Öffnung wird von einem Kunststoffring (3) begrenzt, der mit der Folie nicht fluidleitend verschweißt ist. An den Ring angrenzend befindet sich auf der offenporigen Unterseite aufliegend ein kreisförmiger offenporiger Polyurethanschaum (4) Der Schaum (4) ist fluidleitend mit einem Schlauch (5) verbunden, dieser ist mit einem vakuumerzeugenden System (6) verbunden. Auf der Unterseite der offenporigen Folie sind aus Polyurethanschaumstreifen bestehende Buchstaben und Zahlen (7) aufgelegt. Da die Folie transparent ist, sind sie sichtbar. Sie sind mit der Folie fluidleitend verbunden und kollabieren unter Unterdruckausübung.

Figur 3 b ist ein Querschnitt durch die Folie in Höhe der Buchstaben bzw. Zahlen, die als Streifen der Folie fluidleitend auf die Unterfläche aufgeklebt sind.

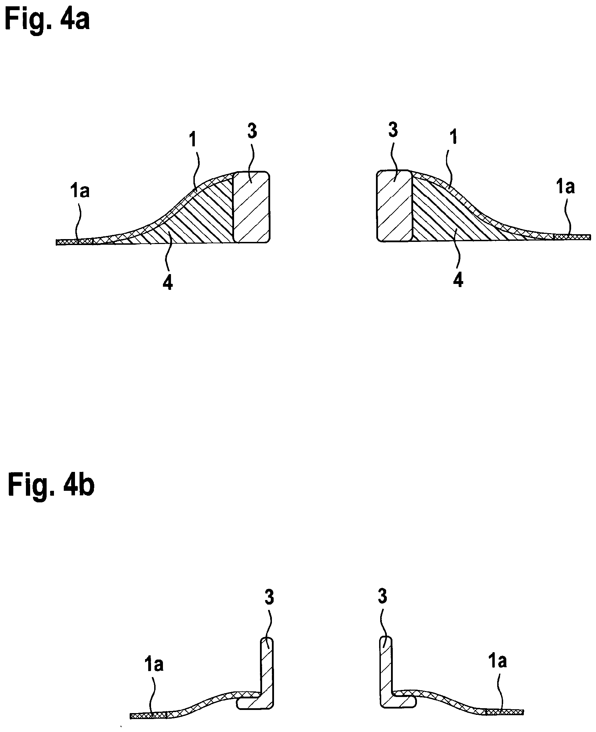

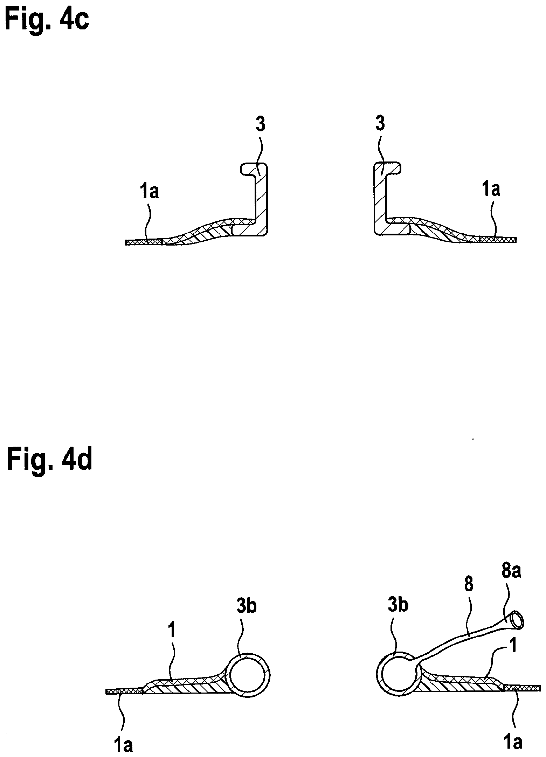

Figuren 4 a/b/c zeigen verschieden Querschnittprofile des massiv gefertigten Kunststoffringes (3). Die einseitig offenporige Folie (1) ist an unterschiedlichen Stellen des Ringes angebracht und unterschiedlich hohe Polyurethanschäume (4) liegen dem Ring an. Der Rand der Folie (1a) ist nicht fluidleitend und kann auf eine Wunde geklebt werden.

In Figur 4a hat der Ring (3) ist ein rechteckiges Profil.

In Figur 4b hat der Ring (3) ein L-förmiges Profil mit schmalen über die Folie (1) hinausragenden Rand. Hierdurch soll das Aufkleben eines Stomabeutels erleichtert werden.

In Figur 4c hat der Ring (3) ein klammerartiges, C-förmiges Profil. Dieses Profil ist wie das Verriegelungsprofil von Stomabeuteln konstruiert, so dass hier direkt ein Beutel aufgedrückt werden könnte.

Figur 4d ist ein Querschnittsprofil eines Kunststoffringes (3b), welcher hohl ist und mit Gas oder Flüssigkeit über einen Kanal (8), welcher mit einem Ventil (8a) ausgestattet ist, befüllt werden kann.

### BEZUGSZEICHENLISTE

- 1: offenporige Folie
- 1a: umlaufender Rand
- 2: Öffnung
- 3: Kunststoffring
- 4: Polyurethanschaum
- 4a / 4b: fingerartige Ausläufer des Polyurethanschaums
- 5: Schlauch
- 5a: Klebefläche
- 5b: Öffnung
- 6: vakuumerzeugendes System
- 7: Markierungselemente
- 8: Kanal
- 8a: Ventil
- 20: Wunde
- 20a: Fistelöffnung
- 20b: Darm

## Patentansprüche

1. Wundversorgungsanordnung, insbesondere zur Versorgung von Wunden im Bereich entero-atmosphärischer Fisteln, mit einer den Durchtritt von Fluiden hemmenden, insbesondere gasdichten Abdeckeinrichtung (1) zum Abdecken der Wunde und/oder eines die Wunde umlaufenden Hautbereichs mit einer Öffnung (2) in der Abdeckeinrichtung (1) sowie einer der Öffnung (2) zugeordneten und fluiddicht mit der Abdeckeinrichtung (1) verbundenen, einer Verformung der Abdeckeinrichtung im Bereich der Öffnung entgegenwirkenden Verstärkungseinrichtung (3, 3b), wobei in einem von der Abdeckeinrichtung (1), der davon abgedeckten Haut bzw. Wunde und der Verstärkungseinrichtung begrenzten Raum ein Unterdruck erzeugt werden kann, **dadurch gekennzeichnet, daß** die Abdeckeinrichtung eine einseitig offenporige Folie (1) aufweist, deren offenporige Seite der Haut zugewandt ist und von einem fluiddicht an die Wunde anschließbaren und fluiddicht damit verbundenen Rand umlaufen wird.

2. Wundversorgungsanordnung nach Anspruch 1, **dadurch gekennzeichnet, daß** die Verstärkungseinrichtung (3) einen Verstärkungsring, insbesondere Kunststoffring, aufweist, an den die Abdeckeinrichtung (1) fluiddicht angeschlossen ist.

3. Wundversorgungsanordnung nach Anspruch 2, **dadurch gekennzeichnet, daß** der Verstärkungsring (3) einen auf die Haut auflegbaren Flanschbereich und einen sich senkrecht dazu, etwa parallel zur Ringachse erstreckenden Stutzenbereich aufweist.

4. Wundversorgungsanordnung nach Anspruch 3, **dadurch gekennzeichnet, daß** der Stutzenbereich an seinem dem Flanschbereich abgewandten Ende einen sich radial nach außen erstreckenden Anschlussflansch aufweist.

5. Wundversorgungsanordnung nach einem der Ansprüche 2 bis 4, **dadurch gekennzeichnet, daß** der Verstärkungsring (3b) zumindest abschnittweise verformbar ausgeführt ist.

6. Wundversorgungsanordnung nach Anspruch 5, **dadurch gekennzeichnet, daß** der Verstärkungsring (3b) zumindest bereichsweise als von einer verformbaren Wand begrenzter Hohlkörper ausgeführt ist, der ggf. über eine Ventilanordnung (8a) mit einem Fluid befüllt werden kann.

7. Wundversorgungsanordnung nach einem der vorhergehenden Ansprüche, **gekennzeichnet durch** eine zwischen der Abdeckeinrichtung (1) und der Haut anzuordnende, nachgiebige und fluidleitende Polsteranordnung (4, 4a, 4b), die die Öffnung (2) zumindest abschnittsweise umläuft und besonders bevorzugt an die Verstärkungseinrichtung (3) angeschlossen ist.

8. Wundversorgungsanordnung nach Anspruch 7, **dadurch gekennzeichnet, daß** die Polsteranordnung (4, 4a, 4b) einen offenporigen Schaum, insbesondere Polyurethanschaum aufweist.

9. Wundversorgungsanordnung nach Anspruch 7 oder 8, **dadurch gekennzeichnet, daß** die Polsteranordnung (4, 4a, 4b) einen die Verstärkungseinrichtung (3, 3b) umlaufenden Polsterring und mindestens einen, vorzugsweise zwei, drei oder mehr sich ausgehend davon radial nach außen erstreckende Polsterarme (4a) aufweist.

10. Wundversorgungsanordnung nach einem der Ansprüche 7 bis 9, **dadurch gekennzeichnet, daß** die Abdeckeinrichtung (1) zumindest abschnittweise durchsichtig ausgeführt ist und die Polsteranordnung (4, 4a, 4b) in einem durchsichtigen Bereich der Abdeckeinrichtung angeordnete buchstaben- und/oder ziffernförmige Markierungselemente (7) aufweist.

11. Wundversorgungsanordnung nach einem der vorhergehenden Ansprüche, **gekennzeichnet durch** eine Einrichtung (6) zum Erzeugen eines Unterdrucks, die über eine Verbindungseinrichtung (5)mit der Abdeckeinrichtung (1) verbunden ist.

12. Wundversorgungsanordnung nach einem der vorhergehenden Ansprüche, **gekennzeichnet durch** eine vorzugsweise lösbar an der Verstärkungseinrichtung (3, 3b) angebrachten, insbesondere beutelförmige Aufnahmeeinrichtung.

## Claims

1. A wound dressing assembly, in particular for dressing wounds in the area of enteroatmospheric fistulae, comprising a covering device (1) which inhibits the passage of fluids and is in particular gas-tight, and covers the wound and/or a skin area surrounding the wound, and is in particular gas-tight, with an opening (2) in the covering device (1) and a reinforcing device (3, 3b) associated with the opening (2) and connected to the covering device (1) in a fluid-tight manner, wherein the reinforcing device counteracts deformation of the covering device in the area of the opening, and wherein a negative pressure can be generated in a space delimited by the covering device (1), the skin or wound covered thereby and the reinforcing device, **characterised in that** the covering device comprises a unilaterally open-celled film (1), the open-celled side of which faces the skin and is surrounded by an edge that is configured to be joined, and is connected, to the wound in a fluid-tight manner.

2. The wound dressing assembly in accordance with claim 1, **characterised in that** the reinforcing device (3) has a reinforcing ring, in particular a plastic ring, to which the covering device (1) is joined in a fluid-tight manner.

3. The wound dressing assembly in accordance with claim 2, **characterised in that** the reinforcing ring (3) has a flange area configured to be placed on the skin and a stub area which extends perpendicular to the flange area, approximately parallel to the ring axis.

4. The wound dressing assembly in accordance with claim 3, **characterised in that** the stub area has, at its end which is facing away from the flange area, a connecting flange which extends radially outwards.

5. The wound dressing assembly in accordance with claims 2 to 4, **characterised in that** at least portions of the reinforcing ring (3b) are deformable.

6. The wound dressing assembly in accordance with claim 5, **characterised in that** at least areas of the reinforcing ring (3b) are a hollow body delimited by a deformable wall, wherein the hollow body can possibly be filled with a fluid by means of a valve arrangement (8a).

7. The wound dressing assembly in accordance with one of the preceding claims, **characterised by** a flexible and fluid-conducting buffer arrangement (4, 4a, 4b), to be arranged between the covering device (1) and the skin, which surrounds at least portions of the opening (2) and particularly preferably is joined to the reinforcing device (3).

8. The wound dressing assembly in accordance with claim 7, **characterised in that** the buffer arrangement (4, 4a, 4b) comprises an open-celled foam, in particular polyurethane foam.

9. The wound dressing assembly in accordance with claim 7 or 8, **characterised in that** the buffer arrangement (4, 4a, 4b) comprises a buffer ring encircling the reinforcing device (3, 3b) and at least one buffer arm (4a), but preferably two, three or more buffer arms, extending radially outwards from the buffer ring.

10. The wound dressing assembly in accordance with claims 7 to 9, **characterised in that** at least portions of the covering device (1) are transparent and the buffer arrangement (4, 4a, 4b) comprises identification elements (7) arranged in a transparent portion of the covering device in the form of letters and/or digits.

11. The wound dressing assembly in accordance with one of the preceding claims, **characterised by** a device (6) for generating negative pressure which is connected to the covering device (1) via a connecting device (5).

12. The wound dressing assembly in accordance with one of the preceding claims, **characterised by** a receiving device, in particular a pouch-shaped receiving device, which is attached, preferably detachably, to the reinforcing device (3, 3b).

## Revendications

1. Système de traitement de plaies, notamment pour le traitement de plaies dans la région de fistules entéro-atmosphériques, comprenant un dispositif de recouvrement (1) empêchant le passage des fluides et notamment étanche aux gaz, destiné à recouvrir la plaie et/ou une région cutanée entourant la plaie, avec une ouverture (2) présente dans le dispositif de recouvrement (1), ainsi qu'un dispositif de renfort (3, 3b) associé à l'ouverture (2) et relié au dispositif de recouvrement (1) de façon étanche aux fluides, agissant contre une déformation du dispositif de recouvrement dans la région de l'ouverture ; une pression négative pouvant être créée dans un espace délimité par le dispositif de recouvrement (1), la peau ou la plaie qu'il recouvre et le dispositif de renfort, **caractérisé en ce que** le dispositif de recouvrement présente une feuille (1) à pores ouverts sur un côté, ledit côté à pores ouverts étant tourné vers la peau et étant entouré d'une bordure pouvant être accolée à la plaie de façon étanche aux fluides et étant reliée à celle-ci de façon étanche aux fluides.

2. Système de traitement de plaies selon la revendication 1, **caractérisé en ce que** le dispositif de renfort (3) présente un anneau de renfort, notamment en plastique, auquel le dispositif de recouvrement (1) est accolé de façon étanche aux fluides.

3. Système de traitement de plaies selon la revendication 2, **caractérisé en ce que** l'anneau de renfort (3) présente une région de bride applicable sur la peau et une région de manchon perpendiculaire à cette dernière et s'étendant sensiblement parallèlement à l'axe de l'anneau.

4. Système de traitement de plaies selon la revendication 3, **caractérisé en ce que** la région de manchon présente, à son extrémité éloignée de la région de bride, une bride de raccord s'étendant radialement vers l'extérieur.

5. Système de traitement de plaies selon l'une des revendications 2 à 4, **caractérisé en ce que** l'anneau de renfort (3b) est réalisé déformable, au moins par sections.

6. Système de traitement de plaies selon la revendication 5, **caractérisé en ce que** l'anneau de renfort (3b) est réalisé, au moins par régions, sous la forme d'un corps creux délimité par une paroi déformable et susceptible, le cas échéant, d'être rempli d'un fluide par le biais d'un dispositif à robinet (8a).

7. Système de traitement de plaies selon l'une quelconque des revendications précédentes, **caractérisé par** un dispositif de rembourrage (4, 4a, 4b) souple et conducteur de fluide, destiné à être disposé entre le dispositif de recouvrement (1) et la peau, lequel entoure l'ouverture (2) au moins par sections et est préférablement accolé au dispositif de renfort (3).

8. Système de traitement de plaies selon la revendication 7, **caractérisé en ce que** le dispositif de rembourrage (4, 4a, 4b) présente une mousse à pores ouverts, notamment une mousse de polyuréthane.

9. Système de traitement de plaies selon la revendication 7 ou 8, **caractérisé en ce que** le dispositif de rembourrage (4, 4a, 4b) présente un anneau de rembourrage entourant le dispositif de renfort (3, 3b) et au moins un, et de préférence deux, trois voire davantage, bras de rembourrage (4a) partant de celui-ci en s'étendant radialement vers l'extérieur.

10. Système de traitement de plaies selon l'une des revendications 7 à 9, **caractérisé en ce que** le dispositif de recouvrement (1) est conçu transparent, au moins par sections, et le dispositif de rembourrage (4, 4a, 4b) présente, dans une région transparente du dispositif de recouvrement, des éléments de marquage (7) sous forme de lettres et/ou de chiffres.

11. Système de traitement de plaies selon l'une quelconque des revendications précédentes, **caractérisé par** un système (6) de production de pression négative raccordé au dispositif de recouvrement (1) par le biais d'un système de raccordement (5).

12. Système de traitement de plaies selon l'une quelconque des revendications précédentes, **caractérisé par** un dispositif de collecte, notamment sous forme d'un sac, agencé sur le dispositif de renfort (3, 3b), de préférence de façon amovible.
